Europäisches Patentamt

**European Patent Office**

**Office européen des brevets**

(11) Numéro de publication: **0 320 382 B1**

(19)

(12) **FASCICULE DE BREVET EUROPEEN**

(45) Date de publication de fascicule du brevet: **11.11.92**  (51) Int. Cl.5: **A61M 5/14**

(21) Numéro de dépôt: **88403110.5**

(22) Date de dépôt: **08.12.88**

(54) **Appareil de perfusion.**

(30) Priorité: **10.12.87 FR 8717440**

(43) Date de publication de la demande:
**14.06.89 Bulletin 89/24**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**US-A- 3 785 683**
**US-A- 4 170 994**
**US-A- 4 173 223**
**US-A- 4 286 948**

(73) Titulaire: **Denis, Jean Roger**
**68, rue François Chénieux**
**F-87000 Limoges(FR)**

Titulaire: **Desnoyers, Pierre Etienne**
**57, avenue Ernest Ruben**
**F-87000 Limoges(FR)**

(72) Inventeur: **Denis, Jean Roger**
**68, rue François Chénieux**
**F-87000 Limoges(FR)**
Inventeur: **Desnoyers, Pierre Etienne**
**57, avenue Ernest Ruben**
**F-87000 Limoges(FR)**

# Description

La présente invention concerne un nouvel appareil de perfusion.

Les dispositifs connus de perfusion sont constitués par une aiguille de raccordement mise en place dans le bouchon de la bouteille qui contient le liquide de perfusion, par un comptegouttes, par un régulateur de débit et par un tube dont l'extrémité comporte un raccord - connu - à une aiguille à perfuser.
Des dispositifs de ce type sont décrits par exemple dans le brevet U.S. 4 173 223.

Ces dispositifs comportent tous, du fait de possibilités de rotation très limitées des éléments les uns par rapport aux autres, des inconvénients connus qui se traduisent essentiellement par des risques de déperfusion et des risques d'écrasement des divers tuyaux souples.
Le brevet U.S. 3 785 683 décrit un dispositif permettant la libre rotation d'un cathéter.
Le brevet U.S. 4 286 948 décrit dans un tout autre domaine un dispositif de traitement des dents utilisant des spirales pour relier différents appareils à un support.

Le dispositif suivant l'invention permet de remédier à ces divers inconvénients ; ce dispositif se caractérise par le fait qu'on introduit au moins une possibilité de rotation entre les divers éléments constitutifs des dispositifs de perfusion connus ; le dispositif selon l'invention est utilisable non seulement en médecine humaine, mais également dans le domaine vétérinaire.

L'invention concerne donc un dispositif servant à perfuser les humains et les animaux comportant d'amont en aval, lorsqu'on se déplace dans le sens du liquide de perfusion, une aiguille de raccordement au récipient contenant ledit liquide, un compte-gouttes, un régulateur de débit et un tube dont l'extrémité comporte un raccord d'aiguille à perfuser, caractérisé en ce que, entre ladite aiguille de raccordement et ledit tube, on dispose d'une pièce agencée de façon que ladite aiguille et ladite pièce puissent être animées d'un mouvement de rotation libre l'une par rapport à l'autre, et que ledit tube est formé en spirale.

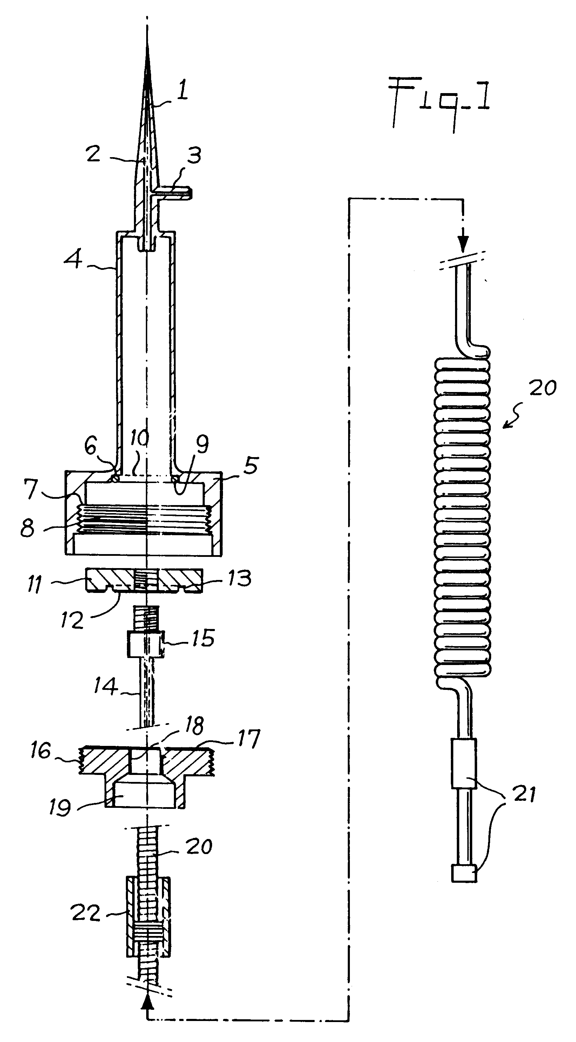

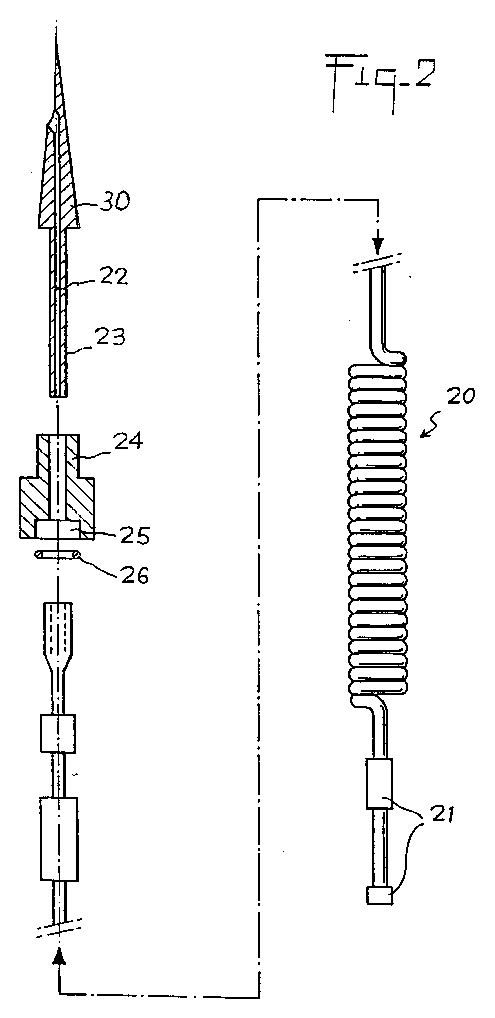

L'invention sera mieux comprise en se référant aux exemples non limitatifs ci-après qui illustrent deux modes de mise en oeuvre de l'invention. Ces exemples sont eux-mêmes illustrés respectivement par les figures 1 et 2.

## Exemple 1

La figure 1 représente en coupe un mode de réalisation de l'invention.

Le dispositif présenté sur la figure 1 comporte une aiguille conique 1 ayant des dimensions de l'ordre de 3,5 cm de hauteur, percée d'un trou 2. A la base de l'aiguille 1, un tube perpendiculaire 3 forme une prise d'air destinée à la décompression du flacon de sérum utilisé avec ce dispositif. Ce raccord 3 est terminé par un petit tube flexible (non représenté) d'environ 20 cm.

En prolongement de la base de l'aiguille 1, se trouve un tube cylindrique 4 en matière transparente. Ce tube compte-gouttes 4 est destiné à vérifier la régulation de la vitesse d'écoulement du sérum par l'observation de la chute des gouttes.

A la base du compte-gouttes 4 et formant une pièce unique moulée, un bouchon à vis 5 comportant différents épaulements intérieurs 6 et 7 ainsi qu'un filetage 8 reçoit différents autres accessoires.

La partie 1-3-4-5 du dispositif peut être constituée en un matériau synthétique, ou autre, en une seule pièce ou en plusieurs assemblées, sans sortir pour cela du cadre de la présente invention.

Dans l'épaulement 6 rentre en force une bague 9 sur laquelle une toile de tamis très fine 10 est tendue.

Ce tamis est destiné à retenir toute impureté qui pourrait provenir du flacon de sérum, par exemple : particules de caoutchouc entraînées lors du perçage du flacon ; les différents types de flacons n'entrent pas dans le cadre de la présente invention.

Dans l'épaulement 7 est disposée une pièce 11 en acier-inox (norme 17/12 MO) recouverte sur la partie 12 d'un revêtement spécial céramique ; une rainure circulaire 13 pratiquée dans le métal et la céramique sert de joint d'étanchéité.

Dans la pièce 11 un tube 14 comporte une partie renflée cylindrique 15 également recouverte d'une couche céramique. Le tube 14 est assemblé à la pièce 11 par filetage ou tout autre procédé connu à ce jour, ou autres procédés à venir.

Terminant cet assemblage, une pièce 16 filetée pour se visser sur le pas de vis 8 est traitée céramique sur sa face 17, ainsi que dans le trou 18. Cette pièce est évidée en 19 pour permettre le passage des tubes 14 et 20.

Les pièces 11-14 et 16 sont rodées ensemble pour que la rotation soit parfaite.

Emboîté sur le raccord inox 14, un tube spiralé 20 en matière synthétique, constitué de 40 spires d'environ 20 mm de diamètre, sert à la liaison avec le raccord d'aiguille 21 ; étant entendu que le nombre ainsi que le diamètre des spires peuvent varier tout en demeurant dans le cadre du présent brevet.

Un régulateur de débit 22 est disposé sur le tube spiralé 20. Le raccord d'aiguille 21 ainsi que le régulateur 22 sont des articles du commerce n'entrant pas dans le cadre de la présente invention.

Le dispositif suivant l'invention évite l'écrasement accidentel du tube 20, ce dernier par ses spirales ne pouvant se trouver coïncé et écrasé sous le patient.

D'autre part, le dispositif de la pièce tournante décrite de 1 à 19 compense la torsion du tube 20 par une rotation d'une partie de ladite pièce évitant tout vrillage du tube et son obturation ; cas que l'on retrouve avec les enfants, les personnes agées, les agités, les animaux en cage.

Les piéces constitutives 11-14 et 16 sont restérilisables, ou éventuellement jetables.

Les pièces 1-4-5 ainsi que le tube 20 et le raccord d'aiguille 21 sont jetables

Les pièces rotatives 11-14 et 16 peuvent être de constitution et revêtement différents sans sortir du cadre de la présente invention.

Exemple 2

La figure 2 représente, en coupe, un autre mode de réalisation de l'invention ; pour des problèmes de stérilisation, il apparaît que cet autre mode de réalisation est le plus intéressant.

Le dispositif présenté sur la figure 2 comporte une aiguille conique 30 percée de part en part d'un trou 22. Cette aiguille est terminée dans sa partie inférieure par un tube droit 23.

Une pièce 24 formant un fourreau vient s'emboîter sur le tube droit 23 en tournant librement sur ce dernier.

A la base de la pièce 24, un logement circulaire 25 est pratiqué qui permet la disposition d'un filtre antimicrobien 26.

A l'extrémité du tube 23, vient se raccorder par emboîtage un tube souple suivi d'un régulateur et d'un compte-gouttes classiques ces deux accessoires n'entrant pas dans le cadre du présent brevet.

A la suite de ce compte-gouttes, est raccordé un tube spiralé 20 suivi d'un embout porte-aiguille 21.

La pièce 24 tourne autour du tube 23 avec un jeu permettant à l'air ambiant de pénétrer dans le flacon après passage au travers du filtre antimicrobien 26 et réaliser ainsi au moins une partie de la prise d'air nécessaire au perfuseur.

On notera que dans cette réalisation :
- il est important qu'entre les pièces 24 et 23 il y ait un certain intervalle laissant passer une certaine quantité d'air ; en effet, ce passage de l'air évite que le liquide se trouvant dans le récipient ne puisse fuir entre l'aiguille et ladite pièce 24,
- compte tenu de ce passage de l'air, il est nécessaire d'intercaler un filtre antimicrobien 26 sur ce trajet de l'air ambiant.

Une différence importante entre les deux modes de réalisation décrits dans ces deux exemples est que, dans le mode de réalisation de l'exemple 1, l'aiguille est fixe par rapport au récipient et la pièce mobile se trouve en aval en 11, alors que, dans l'exemple 2, l'aiguille (avec son tube 23) peut pivoter dans la pièce 24 qui, elle, est fixe par rapport au récipient, car elle est prise dans le bouchon dudit récipient.

**Revendications**

1. Dispositif servant à perfuser les humains et les animaux comportant d'amont en aval, lorsqu'on se déplace dans le sens du liquide de perfusion, une aiguille (1,30) de raccordement au récipient contenant ledit liquide, un compte-gouttes (4), un régulateur de débit (22) et un tube (20) dont l'extrémité comporte un raccord (21) d'aiguille à perfuser, caractérisé en ce que, entre ladite aiguille de raccordement (1) et ledit tube (20), on dispose d'une pièce (11, 24) agencée de façon que ladite aiguille et ladite pièce puissent être animées d'un mouvement de rotation libre l'une par rapport à l'autre, et que ledit tube (20) est formé en spirale.

2. Dispositif selon la revendication (1), caractérisé en ce que ladite aiguille de raccordement (1) est adaptée à être fixe par rapport au récipient contenant le liquide de perfusion et que ladite pièce mobile (11) est une pièce à revêtement céramique placée dans un épaulement situé à l'extrémité du compte-gouttes et comportant un raccord que l'on peut fixer audit tube (20), ladite pièce (11) étant serrée dans ledit épaulement à l'aide d'une pièce, à travers laquelle passe ledit raccord et présentant également sur sa face en contact avec ladite pièce (11), un revêtement de céramique.

3. Dispositif selon la revendication 1, caractérisé en ce que l'aiguille de raccordement (30) est terminée par un tube droit percé (23) passant dans l'axe percé d'une pièce (24) mobile en rotation par rapport à ladite aiguille, mais adaptée à être fixe par rapport au récipient contenant le liquide de perfusion, ledit tube étant raccordé, par l'intermédiaire d'un compte-gouttes et d'un régulateur de débit, audit tube spiralé (20).

4. Dispositif selon la revendication 3, caractérisé en ce que le jeu existant entre le tube (23) et l'axe percé de ladite pièce (24) permet le passage d'une certaine quantité d'air et que l'on dispose d'un filtre antimicrobien sur ce trajet de l'air.

**Claims**

1. Device used to perfuse humans and animals including, from top to bottom, moving in the direction of the perfusion liquid, a connecting needle (1-30) to the receptacle containing said liquid, a dropper (4) a flow regulator (22) and a tube (20) whose end includes a perfusion needle connexion (21) characterized by the fact that between said connecting needle (1) and said tube (20) there is a part (11-24) arranged so that said needle and said part can be brought to rotate, free of each other, and that said tube (20) is in a spiral shape.

2. Device according to the claim (1) characterized by the fact that said connecting needle (1) is adapted to be fixed in relation to the receptacle containing the perfusion liquid and that said mobile part (11) is a part with ceramic covering placed in a collar located at the end of the dropper and including a connexion that can be fitted onto said tube (20) said part (11) being clamped to said collar by means of a part, through which runs said connection and also having on its side in contact with said part (11) a ceramic covering.

3. Device according to claim 1, characterized by the fact that the connecting needle (30) is terminated by a pierced straight tube (23) running in the pierced axis of a part (24) which is mobile during relation to said needle but, adapted to be fixed in relation to the receptacle containing the perfusion liquid, said tube being connected, through means of a dropper and a flow regulator, to said spiral tube (20).

4. Device according to claim 3, characterized by the fact that the space existing between the tube (23) and the pierced axis of said part (24) allows a certain quantity of air to pass and that there is an anti-microbe filter on this air passage

**Patentansprüche**

1. Vorrichtung zur Perfusion von Menschen und Tieren bestehend, in Fließrichtung der Perfusionflüssigkeit, von oben bis unten gesehen, aus einer in Verbindung mit dem die Perfusionsflüssigkeit enthaltenden Behälter stehenden Anschlußnadel (1-30) einen Tropfenzähler (4) einem Mengenregler (22), sowie einem Schlauch (20) dessen Ende mit einem Perfusionsnadelanschluß (21) versehen ist, **dadurch gekennzeichnet,** daß Zwischen der Anschlußnadel (1) und dem Schlauch (20) ein Teil (11-24) angeordnet ist, welches derart ausgebildet ist, daß die Anschlußnadel und das genannte Teil selbst durch eine Drehbewegung unabhängig voneinander bewegt werden können, und daß der Schlauch (20) spiralförmig ausgebildet ist.

2. Vorrichtung gemäß Anspruch (1) **dadurch gekennzeichnet,** daß die Anschlußnadel (1) so ausgebildet ist, daß sie gegenüber dem die Perfusionsflüssigkeit enthaltenden Behälter feststehend ist, und daß das bewegliche Teil (11) ein in einem sich am Ende des Tropfenzählers befindlichen Bund engeordnetes, keramisch beschichtetes und mit einem au dem Schlauch (20) anbringbaren Anschluß versehenes Teil ist, wobei letzteres mittels eines Teils in den Bund eingepreßt ist, durch welches der auf seiner Kontaktseite mit dem Teil (11) ebenfalls keramisch beschichtete Anschluß hindurchführt.

3. Vorrichtung gemäß Anspruch 1, **dadurch gekennzeichnet,** daß Anschlußnadel (30) in einer geraden, durchbohrten Röhre (23) endet, welche durch die durchbohrte Achse eines gegenüber der Nadel drehbaren, jedoch gegenüber dem die Perfusionsflüssigkeit e,thaltenden Behälter feststehend ausgebildeten Teils (24) hindurch führt, wobei die Röhre durch einen Tropfenzähler, sowie einem Mengenregler mit dem spiralförmigen Schlauch (20) verbunden ist.

4. Vorrichtung gemäß Anspruch 3 **dadurch gekennzeichnet,** daß der zwischen der Röhre (23) und der durchbohrten Achse des Teils (24) bestehende Spielraum den Durchtritt einer gewissen Menge Luft ermöglicht, und daß auf diesem Luftweg ein mikrobendichter Filter angeornet ist.

Fig.1

Fig.2